Europäisches Patentamt

European Patent Office

Office européen des brevets

(11) Publication number: **0 450 467 A2**

# EUROPEAN PATENT APPLICATION

(21) Application number: 91104762.9

(51) Int. Cl.5: **C07C 17/24**, B01J 27/125

(22) Date of filing: 26.03.91

(30) Priority: 31.03.90 JP 83000/90

(43) Date of publication of application:
09.10.91 Bulletin 91/41

(84) Designated Contracting States:
AT BE CH DE ES FR GB GR IT LI LU NL SE

(71) Applicant: DUPONT-MITSUI
FLUOROCHEMICALS CO., LTD.
2-3, Otemachi 1-chome
Chiyoda-ku, Tokyo(JP)

(72) Inventor: Okazaki, Susumu
1973-60 Sakato-machi
Mito City, Ibaraki(JP)
Inventor: Ogura, Masatsune
16-11 Arai 1-chome
Ichikawa City, Chiba(JP)
Inventor: Mochizuki, Yasunobu
11-10, Honcho
Fuji City, Shizuoka(JP)

(74) Representative: Hansen, Bernd, Dr.rer.nat. et
al
Hoffmann, Eitle & Partner Patentanwälte
Arabellastrasse 4 Postfach 81 04 20
W-8000 München 81(DE)

(54) Process for isomerization of hydrochlorofluorocarbons.

(57) Hydrochlorofluorocarbons (HCFC) having 2 or 3 carbon atoms can exist in many isomeric forms. Such compounds are useful as replacements for perchlorofluorocarbons as they have a lower destructive effect on the ozone layer. Among them HCFC in which fluorine atoms are spread around the molecule are unstable. This invention provides a process for isomerizing such compounds to HCFC in which the fluorine atoms are clustered together in the molecule.

The isomerization of this invention is carried out by using a fluorinated alumina catalyst of formula $AlF_xO_y$ (in the formula, $0 < x/y \leq 2/3$) and obtained by calcining $Al_2O_3 . n H_2O$ ($n = 0$ to $3$) containing fibrils of polytetrafluoroethylene at a temperature of at least 400°C.

EP 0 450 467 A2

This invention relates to a process for isomerization of hydrochlorofluorocarbons, particularly to a process for isomerization of hydrochlorofluorocarbons to more stable isomers using a fluorinated alumina catalyst.

Among the chlorofluoroalkanes which are commonly named as "flon", perchlorofluorocarbons containing only fluorine and chlorine, and no hydrogen (hereinafter abbreviated as CFC) have been widely used as refrigerant carrier, solvent, foaming agent and so on, as stable and safety substances.

But recently, as CFCs were found to be one of the substances to be the cause of the destruction of the ozone layer and global warming, an international agreement was concluded to restrict the production of CFCs by 2,000 A.D. completely.

Presently, the research and the study of the substances for replacing these CFCs have been investigated. As the most promising replacement compounds, such hydrogen containing chlorofluoroalkane, namely hydrochlorofluorocarbons (hereinafter abbreviated as HCFC), as 1,1-dichloro-2,2,2-trifluoroethane (hereinafter abbreviated as HCFC-123), 1-chloro-1,2,2,2-tetrafluoroethane (hereinafter abbreviated as HCFC-124), and 2-chloro-2,2-difluoroethane (hereinafter abbreviated as HCFC-142b), can be listed.

However, HCFCs can exist in many isomeric forms. For example, dichlorotrifluoroethane has isomers of 1,2-dichloro-1,2,2-trifluoroethane (hereinafter abbreviated as HCFC-123a), 2,2-dichloro-1,1,2-trifluoroethane (hereinafter abbreviated as HCFC-123b) and the like besides the above mentioned HCFC-123.

In the synthesis of HCFC-123, the by-products of these isomers may sometimes be produced and also in the synthesis of HCFC-142b, 2-chloro-1,2-difluoroethane (hereinafter abbreviated as HCFC-142a) and 1-chloro-2,2-difluoroethane (hereinafter abbreviated as HCFC-142) may be sometimes produced as by-products.

Such kinds of HCFC as HCFC-123a, HCFC-123b and 1-chloro-1,1,2,2-tetrafluoroethane (hereinafter abbreviated as HCFC-124a) are more unstable compounds than the HCFC in which the fluorine atoms are clustered together in the molecule such as HCFC-123 and HCFC-124, particularly those HCFC having trifluoromethyl radicals ($CF_3$). For example, as in the case of use as the foaming agent of urethane foam, mixing with other materials, such high reactive HCFC threaten to influence the quality of the product, so it is not preferable. For such application, use of the HCFC of high content of HCFCs which have a clustering of fluorine atoms are desirable. For example, in the HCFC having more than 3 fluorine atoms in the molecule, HCFCs having a trifluoromethyl radical in the molecule, e.g. HCFC-123 and HCFC-124, are the most preferable. And also in dichlorodifluoroethane and chlorodifluoroethane having only 2 fluorine atoms in the molecule, 1,2-dichloro-2,2-difluoroethane (hereinafter abbreviated as HCFC-132b) and HCFC-142b which have difluorochloromethyl radical and have a clustering of halogen atoms in the molecule are more preferable.

However, if the desired HCFC would be purified and separated from the isomer mixture, the boiling point of such isomers are close to each other so that there is the problem that distillation, which is industrially the most generally used separation means, is not economical.

As a method for solving this problem, isomerization of HCFC-123a and the like to HCFC-123 at 100 to 200°C by using catalysts is known. As the catalysts for such an isomerization reaction, for example, alumina catalysts chlorinated and fluorinated by flon, HF and the like have been proposed. However, this method has the defect that the catalytic activity of the catalyst declines quite quickly.

As a method of preventing the decline of the catalytic activity, the method of isomerization by contacting the mixture of perhalogenated carbon and HCFC-123a with the above mentioned chlorinated and fluorinated alumina is known (Japanese patent publication (Kokoku) 27375/1986). This method has the problem that the extra process step of separating HCFC-123 and perhalogenated carbon is needed after the isomerization reaction, and besides, perhalogenated carbon itself that is used as the agent for preventing decline in the catalytic activity is the substance causing the destruction of the ozone layer.

As a result of investigating the catalyst of high activity to isomerization and possessing long life in order to solve the above mentioned problems, the inventors of the present invention have found that a partially fluorinated alumina catalyst having determined component which is obtainable by calcining $Al_2O_3 \cdot n\, H_2O$ (n = 0 to 3) containing fibrils of polytetrafluoroethylene is an excellent catalyst for the isomerization of HCFCs and have thereby accomplished this invention.

That is to say, this invention relates to a process for isomerization of a hydrochlorofluorocarbon comprising 2 or 3 carbon atoms which comprises contacting the hydrochlorofluorocarbon with a fluorinated alumina catalyst of formula $AlF_xO_y$ (wherein $0 < x/y \leq 2/3$) obtainable by calcining $Al_2O_3 \cdot n\, H_2O$ (n = 0 to 3) containing fibrils of polytetrafluoroethylene at a temperature of at least 400°C.

HCFCs used in the isomerization process of this invention are hydrochlorofluorocarbons of 2 to 3 carbon atoms, particularly hydrochlorofluoroethanes containing hydrogen, chlorine and fluorine. The isomerization reaction of HCFCs to isomers in which the halogen atoms are clustered together, particularly with clustering of fluorine atoms, is preceded by converting these HCFCs into a gaseous state, preferably at a temperature of 30 to 300°C, and then contacting the gaseous HCFCs with the above mentioned catalyst. Such isomers have different electro-negativity in the molecule. HCFCs having a trifluoromethyl radical or difluorochloromethyl radical are specific examples of such isomers. As an example of a typical isomerization process,

(1) Isomerization of 1,2-dichloro-1,2,2-trifluoroethane (HCFC-123a) and 2,2-dichloro-1,1,2-trifluoroethane (HCFC-123b) to 1,1-dichloro-2,2,2-trifluoroethane (HCFC-123)

(2) Isomerization of 1-chloro-1,1,2,2-tetrafluoroethane (HCFC-124a) to 1-chloro-1,2,2,2-tetrafluoroethane (HCFC-124).

(3) Isomerization of 1,2-dichloro-1,2-difluoroethane (HCFC-132) and 1,1-dichloro-2,2-difluoroethane (HCFC-132a) to 1,2-dichloro-2,2-difluoroethane (HCFC-132b) and

(4) Isomerization of 2-chloro-1,2-difluoroethane (HCFC-142a) and 1-chloro-2,2-difluoroethane (HCFC-142) to 2-chloro-2,2-difluoroethane (HCFC-142b)

and the like can be listed.

$Al_2O_3 \cdot n\ H_2O$ (n = 0 to 3) which is the starting material from which the catalyst is manufacture, is preferably of high purity. $Al_2O_3 \cdot n\ H_2O$ (n = 0 to 3) can be prepared by various methods although the catalytic activity of the catalyst depends on the preparation method. For example, alumina prepared from aluminium alkoxide or aluminium nitrate can be exemplified as a preferable starting material having high purity. It gives both high catalytic activity and long catalytic life because organic radicals or acid radicals are decomposed and removed by calcining at a temperature of more than 400°C.

On the other hand, alumina containing non volatile acid radicals such as aluminium sulfate and the like shows low catalytic activity and short catalytic life, and alumina containing basic radicals prepared from sodium aluminate and the like has relatively little activity.

The preparation of the catalyst of this invention is characterized in that fibrils of polytetrafluoroethylene are contained in $Al_2O_3 \cdot n\ H_2O$ (n = 0 to 3) of catalytic material and was calcined. When fibrils of polytetrafluoroethylene dispersed homogeneously in alumina are decomposed and removed by calcining, fibrils of polytetrafluoroethylene not only act as a fluorinating agent but also contribute to production of a partially fluorinated catalyst of determined fluoride / oxygen composition having many small holes derived from its fibril structure.

Hence, the fibrils of polytetrafluoroethylene mean polytetrafluoroethylene having fine fibrous structure. Polytetrafluoroethylene is a polymer which is easily fibrillated. Particularly emulsion polymerized polytetrafluoroethylene is easily fibrillated and known to be fibrillated to ultra fine cobweb-type filaments, when a compression-shear action is added by agitation and the like at moderate temperature (Japanese Patent publication (Kokai) 209645/1984).

The preparation process of the fluorinated alumina catalyst is not restricted particularly, but most general process is that at first aluminium compound containing such easily decomposable and removable radicals by calcining as aluminium alkoxide, aluminium nitrate and the like was solved in water, then hydrolized, for example, by aqueous ammonia water to precipitate ammonium hydroxide.

From the precipitate, partially fluorinated alumina catalyst can be obtained by the following methods:

(1) polytetrafluoroethylene of easily fibrillable property is added to the precipitate. Heat compression and shear are added to disperse the fibrils of polytetrafluoroethylene in the precipitate homogeneously, and the mixture is then calcined.

(2) After the precipitate is dried and pulverized to powder, polytetrafluoroethylene of easily fibrillable property is added, allowed with compression-shear strength to disperse the fibrils of polytetrafluoroethylene in the powder homogeneously, and the mixture is then calcined.

(3) The precipitate is dried, further calcined to get alumina. After alumina is pulverized to powder, polytetrafluoroethylene of easily fibrillable property is added and compression-shear strength is allowed to disperse homogeneously the fibrils of polytetrafluoroethylene in the powder, and the mixture is then calcined.

And the like.

In the industrial meaning, process (3) which can utilize commercial alumina is most useful for the preparation of the catalysts for use in the process of this invention.

Addition of polytetrafluoroethylene of easily fibrillable property is preferably more than 0.3 wt% per alumina to $Al_2O_3 \cdot n\ H_2O$ (n = 0 to 3). The upper limit is not restricted but about less than 15 wt% is preferable. The reason is that higher amounts increase the preparation cost of the catalyst and tend to reduce in some degree the catalytic activity. Less than 0.3% of the additional amount is not preferable as the catalytic activity is reduced.

3

The calcining temperature of the catalyst is necessarily more than $400\,^\circ$C as this is the decomposition temperature of polytetrafluoroethylene. The upper limit of the calcining temperature is not necessarily restricted, but excessive temperatures tend to lower the catalytic activity. Accordingly, a temperature less than $1,000\,^\circ$C is desired, particularly $600\,^\circ$C to $900\,^\circ$C is preferable.

The catalyst which is obtained by this process is a partially fluorinated alumina catalyst having a general formula $AlF_xO_y$ (in the formula, $0 < x/y \le 2/3$). Namely, it is necessary that at least part of the catalyst of this invention is fluorinated. Alumina catalysts having no fluorine have insufficient isomerization activity. If fluorination proceeds so that $x/y > 2/3$, the activity again is reduced.

Fluorine content by xps (x-ray photoelectron spectrum analysis) is within the range of about 1 to 33 mol%, particularly the range of fluorine content in the catalyst with high catalytic activity is about 2 to 10%. As for the reaction condition of the isomerization of HCFC of this invention, the temperature may suitably be higher than the boiling point of HCFC of the reactant but lower than the decomposition temperature (about $300\,^\circ$C). Comparing with the optimum reaction temperature range (about 100 to $150\,^\circ$C) of the hitherto known partially fluorinated alumina catalyst treated by flons, the catalytic activity of the catalyst used in the present invention is higher and the optimum reaction temperature is lower and in the range of 30 to $100\,^\circ$C which is lower by more than $50\,^\circ$C. And also as a result of this, the catalytic life can be increased by more than 3 fold.

[Examples]

Example 1.

To 1.7 kg of the pure water heated at $100\,^\circ$C, 70g of aluminium isopropoxide was added agitatingly and hydrolyzed for 3 hours. After leaving to stand for one day, they were rinsed and filtered to get filter cake (aluminium hydroxide). Then, the filter cake was removed into mortar. 0.875g of polytetrafluoroethylene of easy fibrillability ("Teflon" K10-J made at DuPont-Mitsui fluorochemicals Co., Ltd.) was added and mixed for about 3 minutes and dried for one hour at about $100\,^\circ$C. Then, polytetrafluoroethylene of easy fibrillability was kneaded to be untwisted, keeping whole filter cake in the wet and warm condition, and was fibrillated. After that, kneading and drying was repeated 3 to 4 times for 60 minutes.

The filter cake containing thus obtained polytetrafluoroethylene fibrils was dried for 1 day at $120\,^\circ$C, and pulverized to the powder of uniform 22 to 40 mesh. 1,2 g of them was put into reactor tube and calcined for 3 hours at $600\,^\circ$C in $N_2$ stream to get fluorinated alumina catalyst.

The element analysis values of aluminium, fluorine and oxygen in the catalyst by x-ray photoelectron spectrum analysis were shown as Table 1.

Then, by using HCFC-123 containing 10% of HCFC-123a as the raw material of the isomerization, HCFC-123a was isomerized to HCFC-123 at 120 ml/min(gas) of the feeding rate of the raw material and $60\,^\circ$C of the reaction temperature by the above-mentioned catalyst. The conversion rate of the isomerization of HCFC-123a to HCFC-123 after 1 hour and 3 hours are shown in Table 2.

Example 2.

The isomerization reaction was carried out in the same condition as Example 1 except varying the reaction temperature at $40\,^\circ$C by using the catalyst of the example 1.

The results were shown in Table 2.

Example 3.

Except varying the additional amount 0.35 g of polytetrafluoroethylene of easy fibrillability at same condition as in Example 1, fluorinated alumina catalyst was prepared. The element analysis values of the catalyst were shown in Table 1.

By using this catalyst, except varying the reaction temperature to $80\,^\circ$C, the isomerization reaction was carried out in the same condition as in Example 1. The results were shown together in table 2.

Example 4.

Alumina catalyst (N611N made at Nikki Kagaku Co) was pulverized, added with 0.35g of polytetrafluoroethylene of easily fibrillable property, mixed for nearly 3 minutes and heated for 30 minutes at about $80\,^\circ$C. In the warm condition, polytetrafluoroethylene of easily fibrillable property was kneaded to be untwisted and fibrillated. 1.2g of them was put into reaction tube and calcined for 3 hours at 600°C in $N_2$ stream to obtain fluorinated alumina catalyst. The element analysis values of the catalyst were shown in Table 1.

By using HCFC-123 containing 10% of HCFC-123a as the raw material of the isomerization reaction, HCFC-123a was isomerized to HCFC-123 at 120ml/min(gas) of the feed rate of the raw material at 80°C of the reaction temperature by the use of this catalysts.

The result was shown in table 2

Comparative example 1.

Into 1.7kg of pure water heated at 100°C, 70g of aluminium isopropoxide was added agitatingly and hydrolyzed for 3 hours. After leaving to stand,for one day,it was rinsed and filtered to obtain filter coke (aluminium hydroxide). Then, it was dried for 1 day at 120°C and pulverized to the powders of uniform 22 to 40 mesh. 1.2g among them were put into reactor tube and calcined for 3 hours at 600°C in $N_2$ stream to obtain alumina catalyst.

As the raw material of the isomerization reaction, HCFC-123 containing 10% of HCFC-123a was used. By the use of this catalyst, HCFC-123a was isomerized to HCFC-123 at 120ml/min(gas) of the feed rate of the raw materials at 60°C of the reaction temperature.

The result was shown in table 2.

Comparative example 2

Alumina catalyst (Made at Nikki Kagaku Co. N611N) was pulverized to the powders of uniform 22 to 40 mesh. 1.2g among them was put into reactor tube and calcined for 3 hours at 600°C in $N_2$ stream to obtain catalyst.

By using HCFC-123 containing 10% of HCFC-123a as the raw material of the isomerization reaction, by this catalyst, HCFC-123a was isomerized to HCFC-123 at 120ml/min(gas) of the feed rate of the raw materials at 60°C of the reaction temperature.

The result was shown in Table 2.

Comparative example 3

Alumina catalyst (Made at Nikki Kagaku Co. N611N) was pulverized to the powders of uniform 22 to 40 mesh. 1.2g among them was put into reactor tube, calcined for 3 hours at 600°C in $N_2$ stream and then introduced with the mixing gas of CFC-13/$N_2$ (12/88 vol%) at the rate of 150 ml/min to obtain partially fluorinated alumina catalysts activated for 10min at 420°C. The element analysis values of the catalyst by x-ray photoelectron spectrum analysis were shown in table 1. Comparing with the catalyst of example 1,2 and example 3,4, the degree of the fluorination is more advanced. It is the catalyst containing small amount of chlorine.

By using HCFC-123 containing 10% of HCFC-123a as the raw material of the isomerization reaction by this catalyst. HCFC-123a was isomerized to HCFC-123 at the feed rate of the raw materials of 120 ml/min-(gas) at the reaction temperature of 120°C.

The result was shown in table 2.

Comparative example 4

As alumina catalyst containing alkali metal, N611A made at Nikki Kagaku Co was used. In the same method as in comparative example 3 calcination and activation were carried out to obtain partially fluorinated catalysts.

By using HCFC-123 containing 10% of HCFC-123a as the raw material of isomerization reaction, HCFC-123a was isomerized to HCFC-123 at the feed rate of the raw materials of 120 ml/min(gas) at the reaction temperature of 120°C.

The results are shown in table 2.

Table 1. Element analysis values of the catalyst.

Element analysis values

|  | Example 1,2 | Example 3,4 | Comparative Example 3 |
|---|---|---|---|
| Al | 35.5%(1) | 37.3%(1) | 32.4%(1) |
| F | 8.3%(0.2) | 3.9%(0.1) | 23.2%(0.7) |
| O | 56.2%(1.6) | 58.8%(1.6) | 44.2%(1.4) |
| Cl |  |  | 0.2%(0.1) |

Note: ( ) show the element ratio of the content.

Table 2. The conversion rate of the isomerization of HCFC-123a to HCFC-123.

|  | Conversion rate of isomerization reaction % | |
|---|---|---|
|  | After 1 hour | After 3 hours |
| Example 1 | 99.9 | 85.0 |
| Example 2 | 95.0 | 88.0 |
| Example 3 | 99.5 | 80.0 |
| Example 4 | 98.0 | 65.0 |
| Comparative Example 1 | 3.5 | 0.0 |
| Comparative Example 2 | 35.5 | 4.0 |
| Comparative Example 3 | 85.0 | 0.0 |
| Comparative Example 4 | 0.3 | 0.0 |

As clarified from the result of table 2, in the isomerization reaction by using the catalyst of this invention, conversion rate of the isomerization of HCFC-123a to HCFC-123 is higher and furthermore the declining of the catalytic activity after aging is smaller. On the contrary, in the comparative example 1 and comparative example 2 using alumina catalyst, the activity is extraordinarily low. In the case of using alumina catalyst chloro-fluorinated by flon, depending on alumina used as a raw material, pretty higher level of initial activity can be obtained but activity declines rapidly with the passage of time.

According to the present invention, using partially fluorinated alumina catalyst obtained by calcining $Al_2O_3$ n $H_2O$ (n = 0 to 3) containing fibrils of polytetrafluoroethylene as catalyst, HCFCs can be isomerized at high conversion rate, furthermore the catalyst life is longer and HCFC of high utilization values can be obtained as the substitution for CFC. So that the contribution to solve the global environmental problems caused by flon is exceedingly great.

## Claims

1. A process for isomerization of a hydrochlorofluorocarbon comprising two or three carbon atoms, which comprises contacting the hydrochlorofluorocarbon with a fluorinated alumina catalyst of formula $AlF_xO_y$ (wherein $0 < x/y \leq 2/3$) obtainable by calcining $Al_2O_3 \cdot n\ H_2O$ ($n = 0$ to $3$) containing fibrils of polytetrafluoroethylene at a temperature of at least $400\,^\circ C$.

2. The isomerization process according to claim 1 wherein the hydrochlorofluorocarbon is a hydrochlorofluoroethane.

3. The isomerization process according to claim 2 which comprises isomerizing 1,2-dichloro-1,2,2-trifluoroethane (HCFC-123a) and/or 2,2-dichloro-1,1,2-trifluoroethane (HCFC-123b) to 1,1-dichloro-2,2,2-trifluoroethane (HCFC-123).

4. The isomerization process according to claim 2 which comprises isomerizing 1-chloro-1,1,2,2-tetrafluoroethane (HCFC-124a) to 1-chloro-1,2,2,2-tetrafluoroethane (HCFC-124).

5. The isomerization process according to claim 2 which comprises isomerizing 1,2-dichloro-1,2-difluoroethane (HCFC-132) and/or 1,1-dichloro-2,2-difluoroethane (HCFC-132a) to 1,2-dichloro-2,2-difluoroethane (HCFC-132b).

6. The isomerization process according to claim 2 which comprises isomerizing 2-chloro-1,2-difluoroethane (HCFC-142a) and/or 1-chloro-2,2-difluoroethane (HCFC-142) to 2-chloro-2,2-difluoroethane (HCFC-142b).

7. The isomerization process according to any preceding claim, characterized in that $Al_2O \cdot n\ H_2O$ ($n = 0$ to $3$) containing fibrils of polytetrafluoroethylene is calcined at a temperature of $600\,^\circ C$ to $900\,^\circ C$.

8. The isomerization process according to any preceding claim, characterized in that the isomerization reaction is carried out at a temperature of 30 to $300\,^\circ C$.

9. A fluorinated alumina compound of formula $AlF_xO_y$ (wherein $0 < x/y \leq 2/3$) obtainable by calcining $Al_2O_3 \cdot n\ H_2O$ ($n = 0$ to $3$) containing fibrils of polytetrafluoroethylene at a temperature of at least $400\,^\circ C$.